# EUROPEAN PATENT SPECIFICATION

(11) **EP 2 869 750 B1**
(45) Date of publication and mention of the grant of the patent: **07.11.2018**
(21) Application number: 13740119.6
(22) Date of filing: 08.07.2013
(51) Int. Cl.: A61B 1/005, A61B 1/233

(54) **SINUS ENDOSCOPE**
SINUSENDOSKOP
ENDOSCOPE DE SINUS

(30) Priority: 09.07.2012 US 201213544681
(43) Date of publication of application: 13.05.2015
(73) Proprietor: Gyrus Acmi Inc., Southborough, MA 01772 (US)
(72) Inventor: KONSTORUM, Gregory, Stamford CT (US)
(74) Representative: Manitz Finsterwald Patentanwälte PartmbB
(86) International application number: PCT/US2013/049563
(87) International publication number: WO 2014/011538

(56) References cited:
- US-A1- 2007 249 899
- US-A1- 2007 293 726
- US-A1- 2008 249 483
- US-A1- 2009 171 159
- US-A1- 2010 030 031
- US-A1- 2010 099 946
- US-A1- 2011 004 057

## Description

### CROSS-REFERENCE TO RELATED APPLICATIONS

This application is a continuation of United States Application No. 13/544,681 filed on July 9, 2012.

### FIELD

The present disclosure relates to a medical instrument, and more particularly, to an endoscope having a working channel, the endoscope being configured to be inserted into a nostril and a sinus.

### BACKGROUND

The statements in this section merely provide background information related to the present disclosure and may or may not constitute prior art.

Endoscopes are used by medical professionals for insertion to a body cavity for various diagnostic and medical treatment procedures. For example, to treat a sinus, an endoscope is inserted into a nostril and advanced into or near the sinus cavity. Once the sinus endoscope is positioned in the desired location, a treatment instrument can be advanced through the working channel of the sinus endoscope into the sinus.

Because the sinus cavity is close to the nostril opening through which is it reached, a relatively short endoscope is used for insertion into a sinus. As such, twisting or contorting of the endoscope to rotate the distal end of the endoscope and/or the treatment instrument may be problematic, because endoscope is not long enough to easily endure the contortion loads placed thereon from twisting and/or contorting the sinus endoscope. Therefore, it is desired to have a sinus endoscope that can be rotated and/or deflected as needed, without placing undesirable torsional loads on the endoscope.

Conventional endoscopes are described in documents US 2011 / 0 004 057 A1, US 2007 / 0 249 899 A1, US 2010 / 0 030 031 A1, US 2010 / 0 099 946 A1, US 2007 / 0 293 726 A1, US 2008 / 0 249 483 A1 and US 2009 / 0 171 159 A1.

### SUMMARY

In one aspect, the present disclosure provides an endoscope for insertion into a small body cavity like, or for example, a nostril and a sinus. The endoscope includes a control section, an insertion tube with a working channel tube, and a grip portion. The control section has portions defining a working channel port. The insertion tube extends from the control section, and the insertion tube has a first section and a second section. The first section defines a longitudinal axis and a first lumen extending through the first section. The first section has a proximal end connected to the control section. The second section defines a second lumen extending through proximal and distal ends of the second section. The proximal end of the second section is connected to a distal end of the first section. The second section is bendable with respect to the first section. The second lumen is in communication with the first lumen.

The working channel tube is disposed within the first and second lumens, the working channel tube defines a working lumen extending through proximal and a distal ends of the working channel tube. The working lumen is in communication with the working channel port. The grip portion is connected to the control section, wherein the insertion tube and the control section are rotatable with respect to the grip portion. The second section is formed of shape memory alloy, wherein the second section includes a passive deflection section and an active deflection section, the passive deflection section being connected to and extending from the distal end of the first section, the active deflection section extending from the passive deflection section to the distal end of the second section. The endoscope further comprises a wire sheath disposed within the first and second lumens, the wire sheath having an interior region and being made of shape memory alloy. The endoscope further comprises a single control cable attached to the distal end of the second section of the insertion tube, only the single control cable extending through the interior region of the wire sheath, the control cable being operable to deflect the active deflection section but not the passive deflection section when the control cable is pulled.

In another aspect, which may be combined with or separate from other aspects described herein, an endoscope is provided for insertion into a small body cavity like, or for example, a nostril and a sinus. The endoscope includes a control section, an intermediate part, a shaft bushing, an insertion tube, a working channel tube, and a grip portion. The control section has portions defining a working channel port. The intermediate part is connected to the control section by at least one intermediate pin. The shaft bushing is connected to the intermediate part by at least one shaft pin. The insertion tube extends from the shaft bushing. The insertion tube has a rigid tube section and a flexible tube section. The rigid tube section defines a longitudinal axis and a first lumen extending through the rigid tube section. The rigid tube section has a proximal end connected to the shaft bushing. The flexible tube section defines a flexible tube lumen extending through proximal and distal ends of the flexible tube section. The proximal end of the flexible tube section is connected to a distal end of the rigid tube section. The flexible tube section is bendable with respect to the rigid tube section. The flexible tube lumen is in communication with the rigid tube lumen.

The working channel tube is disposed within the flexible tube lumen and the rigid tube lumen. The working channel tube defines a working tube lumen extending through proximal and distal ends of the working channel tube. The proximal end of the working channel tube is connected to the control section. The working lumen is in communication with the working channel port. The grip portion is connected to the control section, wherein the insertion tube and the control section are rotatable with respect to the grip portion. The flexible tube section is formed of shape memory alloy, wherein the flexible tube section includes a passive deflection section and an active deflection section, the passive deflection section being connected to and extending from the distal end of the rigid tube section, the active deflection section extending from the passive deflection section to the distal end of the flexible tube section. The endoscope further comprises a wire sheath disposed within the flexible tube lumen and the rigid tube lumen, the wire sheath having an interior region and being made of shape memory alloy. The endoscope further comprises a single control cable attached to the distal end of the flexible tube section of the insertion tube, only the single control cable extending through the interior region of the wire sheath, the control cable being operable to deflect the active deflection section but not the passive deflection section when the control cable is pulled.

Accordingly, pursuant to one aspect of the present invention, there is contemplated an endoscope for insertion into a small body cavity like a nostril and a sinus, the endoscope comprising one or more of the following: a control section having portions defining a working channel port; an insertion tube extending from the control section, the insertion tube having a first section and a second section, the first section defining a longitudinal axis and defining a first lumen extending through the first section, the first section having a proximal end connected to the control section, the second section defining a second lumen extending through proximal and distal ends of the second section, the proximal end of the second section being connected to a distal end of the first section, the second section being bendable with respect to the first section, the second lumen being in communication with the first lumen; a working channel tube disposed within the first and second lumens, the working channel tube defining a working lumen extending through proximal and a distal ends of the working channel tube, the working lumen being in communication with the working channel port; and a grip portion connected to the control section, wherein the insertion tube and the control section are rotatable with respect to the grip portion.

Accordingly, pursuant to another aspect of the present invention, there is contemplated an endoscope for insertion into a small body cavity like a nostril and a sinus, the endoscope comprising one or more of the following: a control section having portions defining a working channel port; an intermediate part connected to the control section by at least one intermediate pin; a shaft bushing connected to the intermediate part by at least one shaft pin; an insertion tube extending from the shaft bushing, the insertion tube having a rigid tube section and a flexible tube section, the rigid tube section defining a longitudinal axis and defining a first lumen extending through the rigid tube section, the rigid tube section having a proximal end connected to the shaft bushing, the flexible tube section defining a flexible tube lumen extending through proximal and distal ends of the flexible tube section, the proximal end of the flexible tube section being connected to a distal end of the rigid tube section, the flexible tube section being bendable with respect to the rigid tube section, the flexible tube lumen being in communication with the rigid tube lumen; a working channel tube disposed within the flexible tube lumen and the rigid tube lumen, the working channel tube defining a working tube lumen extending through proximal and distal ends of the working channel tube, the proximal end of the working channel tube being connected to the control section, the working lumen being in communication with the working channel port; and a grip portion connected to the control section, wherein the insertion tube and the control section are rotatable with respect to the grip portion.

The invention may be further characterized by one or any combination of the features described herein, such as: the insertion tube and the control section are rotatable with respect to the grip portion in a first direction about ninety degrees from a home position and in a second direction about ninety degrees from the home position, the first direction being opposite the second direction; the first section of the insertion tube comprises a rigid tube and the second section of the insertion tube comprises a flexible tube; the distal end of the second section is configured to be bent from a first position that is located at a first angle from the longitudinal axis to a second position that is located at a second angle from the longitudinal axis, the first and second angles being located on opposite sides of the longitudinal axis, the first angle being at least 30 degrees and the second angle being at least 110 degrees, the second section being bendable along a plane between the first angle and the second angle; the second section is formed of shape memory alloy; the second section is preformed into a preformed curved shape, wherein the distal end of the second section extends at an angle of at least 30 degrees from the longitudinal axis in the preformed curved shape; a plurality of pins coupling the control section to the insertion tube; the working channel tube has an inner diameter of at least 0.4 millimeters; a control cable attached to the distal end of the second section of the insertion tube, the control cable extending through the first and second lumens, the control cable being operable to bend the second section when the control cable is pulled; an illumination bundle extending through the first and second lumens; an imaging bundle extending through the first and second lumens; a lens disposed at a proximal end of the grip portion; and the first section of the insertion tube has a length of about 160-170 millimeters and the second section of the insertion tube has a length of about 60-67 millimeters.

Further aspects, advantages and areas of applicability will become apparent from the description provided herein. It should be understood that the description and specific examples are intended for purposes of illustration only and are not intended to limit the scope of the present disclosure.

### DRAWINGS

The drawings described herein are for illustration purposes only and are not intended to limit the scope of the present disclosure in any way.
FIG. 1 is a perspective view of an endoscope in accordance with the principles of the present disclosure;
FIG. 2 is a partially cut away side view of the endoscope of FIG. 1, according to the principles of the present disclosure;
FIG. 3 is a is a close-up partially cut away side view of a control section of the endoscope of FIGS. 1-2, in accordance with the principles of the present disclosure;
FIG. 4 is a partially cut away perspective view (25% cut away) of the control section of the endoscope of FIGS. 1-3, according to the principles of the present disclosure; and
FIG. 5 is a cross-sectional view of the endoscope of FIGS. 1-4, taken along the line 5-5 of FIG. 2, in accordance with the principles of the present disclosure.

### DETAILED DESCRIPTION

The following description is merely exemplary in nature and is not intended to limit the present disclosure, application, or uses.

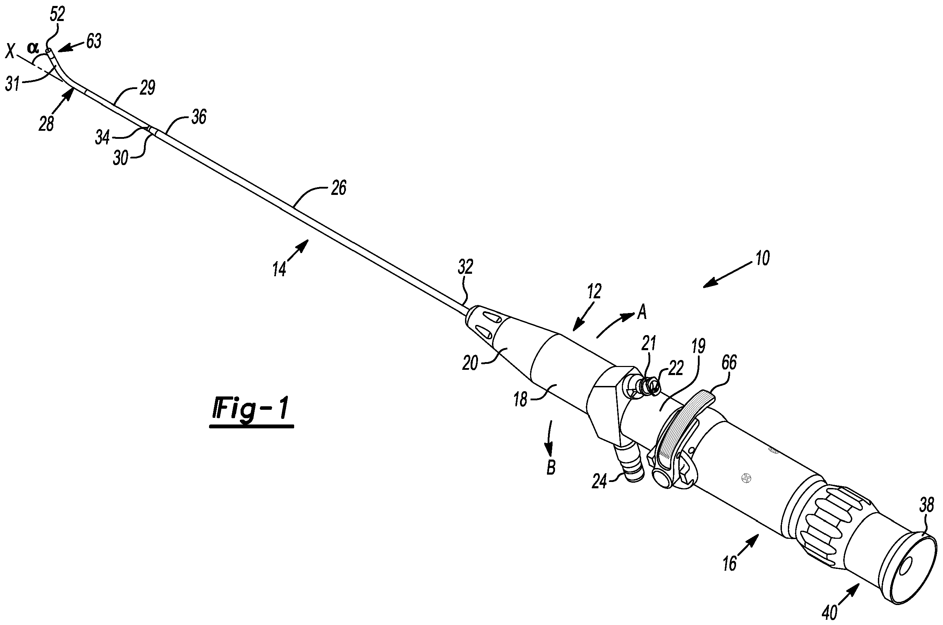

With reference to the figures, wherein like numerals indicate like components, and specifically with reference to FIG. 1, an example of an endoscope in accordance with the principles of the present disclosure is illustrated and generally designated at 10. The endoscope 10 is intended for use in a small body cavity such as a sinus and/or nostril. However, in alternate embodiments, the endoscope 10 could be used for insertion into other body cavities.

The endoscope 10 has a control section 12, an insertion tube 14, and a handle or grip portion 16. The control section 12 has a generally circular cylindrical portion 18 connected to a conical portion 20, however it should be understood that any shape could be used. In the illustrated embodiment, the cylindrical portion 18 is unitarily formed with the conical portion 20. The control section 12 has portions with a working channel port 22. The working channel port 22 is formed through a boss 21 that is attached to a base portion 19 of the control section 12, by way of example. The base portion 19 has a generally circular cylindrical shape. The working channel port 22 is a generally circular opening through the control section 12, although other shapes could be used without falling beyond the spirit and scope of the present disclosure. More particularly, the working channel port 22 is a generally circular cylindrical opening through the boss 19 that protrudes from the base portion 19 of the control section 12. The working channel port 22 is used to insert an instrument through the endoscope 10 and into a body cavity of a patient. The control section 12 also has light source post 24, in this example, for connecting a light source (not shown).

The insertion tube 14 is connected to and extends from the control section 12. The insertion tube 14 has a generally circular cylindrical shape, in this embodiment. The insertion tube 14 includes a first section 26, which may be rigid or semi-rigid, and a second section 28, which may be flexible, bendable, and/or malleable.

The first section 26 defines a longitudinal axis X of the endoscope 10. The first section 26 has a proximal end 32 connected to the conical portion 20 of the control section 12. The first and second sections 26, 28 are connected to each other at a conical part 30. The conical part 30 is optional. For example, the conical part 30 could be eliminated. Thus, the second section 28 has a proximal end 34 that is connected to a distal end 36 of the first section 26, either through the conical part 30 or directly. The second section 28 is bendable with respect to the first section 26. In the illustrated embodiment, the second section 28 has a smaller outer diameter than the diameter of the first section 26.

The second section 28 may have two parts: a passive deflection section 29 and an active deflection section 31. The passive deflection section 29 is connected to the conical part 30 and the active deflection section 31 and extends from the passive deflection section 29. Thus, the passive deflection section 29 extends between the conical part 30 and the active deflection section 31. The active deflection section 31 extends serially from the passive deflection section 29 to a distal end 52 of the second section 28.

The grip portion 16 is connected, such as by a rotatable connection, to the control section 12. The grip portion 16 has a generally circular cylindrical shape. An ocular lens 38 for viewing through the endoscope 10 may be included on a proximal end 40 of the grip portion 16. The grip portion 16 is a handle for use by a medical professional to hold the endoscope 10. The insertion tube 14 and the control section 12 are rotatable with respect to the grip portion 16. In other words, the grip portion 16 may remain substantially stationary (for example, in the hand of a medical professional), while the control section 12 is rotated with respect to the grip portion 16. The insertion tube 14 is rotationally fixed to the control section 12, and therefore, the insertion tube 14 rotates when the control section 12 is rotated. In one embodiment, the insertion tube 14 and the control section 12 are rotatable with respect to the grip portion 16 in a first direction A about ninety degrees from a home position and in a second direction B about ninety degrees from the home position, wherein the first direction A is opposite the second direction B.

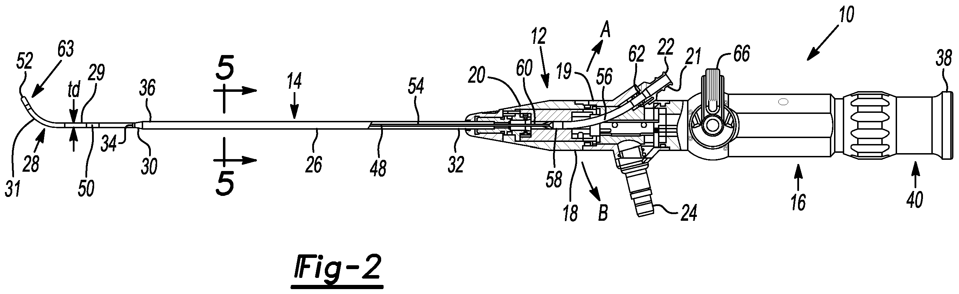

Referring now to FIGS. 1-4, the insertion tube 14 will now be described in greater detail. Like numerals indicate like components in the several figures. The first section 26 of the insertion tube defines a first lumen 48 extending through itself. The second section defines a second lumen 50 extending through itself. The first lumen 48 extends through the proximal and distal ends 32, 36 of the first section 26 and along the length of the first section 26. Likewise, the second lumen 50 extends through the proximal end 34 and a distal end 52 of the second section 28 and along the length of the second section 28. The second lumen 50 is in communication with the first lumen 48.

A working channel tube 54 is disposed within the first and second lumens 48, 50. The working channel tube 54 defines a working lumen 56 extending through proximal and distal ends 62, 63 of the working channel tube 54, and along the length of the working channel tube 54. In the illustrated embodiment, the working channel tube 54 has a wide part 58 disposed within the control section 12, and a thin part 60 connected to, and in communication with, the wide part 58. The thin part 60 is connected to the wide part 58 within the control section 12, and the thin part 60 extends into the insertion tube 14, from the proximal end 32 of the first section 26 to the distal end 52 of the second section 28 of the insertion tube 14. The proximal end 62 of the working channel tube 54 is connected to the portions of the control section 12 forming the working channel port 22. The working lumen 56 is in communication with the working channel port 22 at the proximal end 62 of the working channel tube 54. Accordingly, a working device (not shown) may be inserted into the working channel tube 54 through the working channel port 22, and such devices may be advanced through the working channel tube and out of the distal end 52 of the second section 28 of the insertion tube 14.

As described above, the first section 26 of the insertion tube 14 comprises a rigid tube, and the second section 28 of the insertion tube 14 comprises a flexible tube. The distal end 52 of the second section 28 is configured to be bent in an angular direction, for example, at an angle α, from the longitudinal axis X of the insertion tube 14. In other words, the second section 28 is flexible and may be deflected away from the longitudinal axis X. The distal end 52 of the second section 28 may be bent from a first position that is located at a first angle from the longitudinal axis X to a second position that is located at a second angle from the longitudinal axis X, where the first and second angles are located on opposite sides of the longitudinal axis X within a single plane, by way of example. In one variation, the first angle is at least 30 degrees and the second angle is at least 110 degrees. Thus, the second section 28 is deflectable from about -30° to about +110°. The second section 28 is bendable along a single plane between the first angle and the second angle, by way of example.

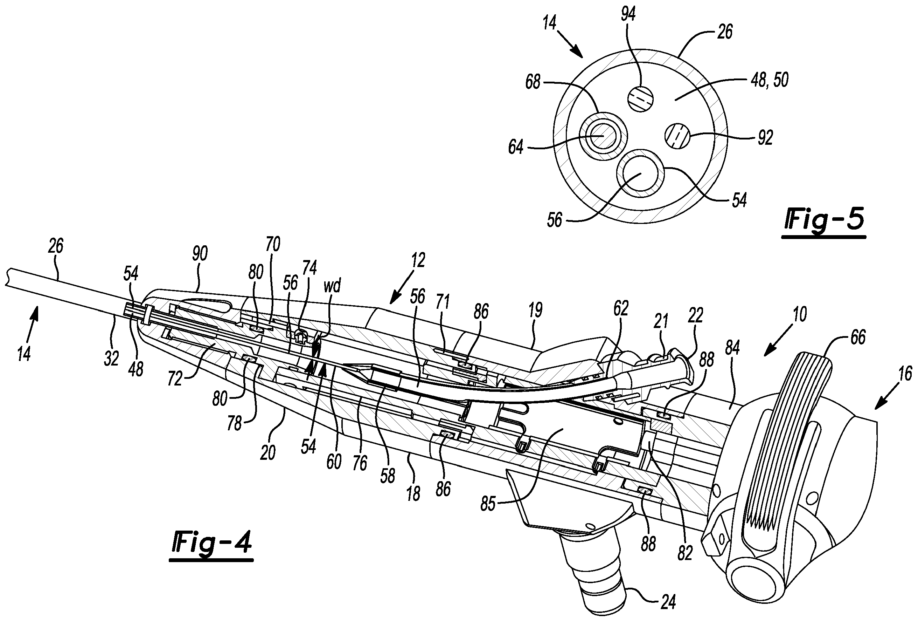

To deflect the distal end 52 of the second section 28, one or more control wires or cables 64, such as a pull cable (shown in cross-section in FIG. 5), may be attached to the distal end of the second section 28 and pulled. In the illustrated embodiment, a single control cable 64 is employed with no additional control cables in the insertion tube 14.

In one example, the section second 28 may be formed of a superelastic material such as a shape memory material that is normally bent at about -30° with respect to the longitudinal axis X (with no pulling of the control cable 64). The shape memory material could include, for example, a shape memory alloy such as Nitinol or Tinel. The shape memory material may be configured to return to its original position after being deflected. In one variation, the second section 28 may be preformed into a preformed curved shape, wherein the distal end 52 of the second section 28 extends at an angle of at least or about 30° from the longitudinal axis X in the preformed curved shape. As the control cable 64 is pulled, the distal end 52 can be pulled from -30° to 0°, and from 0° to +110°, by way of example, along a single plane.

A deflection control lever 66 located on the grip portion 16 may be used to pull the control cable 64, by way of example. The deflection control lever 66 is, or is connected to, an actuator that pulls or moves the control cable 64 to deflect the active deflection section 31 of the second section 28, but not the passive deflection section 29, in this variation. Thus, the deflection control lever 66 is configured to be moved by the user, which results in pulling or releasing the control cable 64 to deflect the active deflection section 31 of the control cable 64. The actuator may be a drum or pulley rotatably connected to the grip portion 16, or the actuator could be any other suitable device, such as a rocker arm or a knob. The control cable 64 extends from the actuator, through the control section 12 and the insertion tube 14, to the distal end 52 of the insertion tube 14. The control cable 64 is attached to the distal end 52. Thus, the control cable 64 is operable to bend the second section 30 when the control cable 64 is pulled or released.

The control cable 64 may pass through the control section 12 and the insertion tube 14 in a wire sheath 68, which has a generally cylindrical tube shape. The wire sheath 68 may be made of shape memory material, such as Nitinol or Tinel, by way of example.

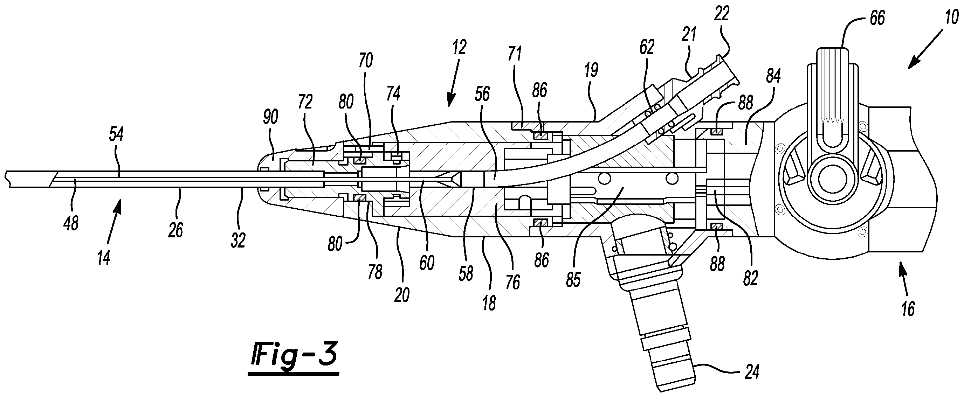

Referring now to FIGS. 3-4, additional optional details of the control section 12 are illustrated. A plurality of intermediate pins 70 couple the control section 12 to the insertion tube 14. More specifically, the intermediate pins 70 fix the conical portion 20 of the control section 12, which may be an intermediate part of the assembly 10, to a shaft bushing 72 that is fixedly connected to the insertion tube 14. A first O-ring 80 is disposed between the shaft bushing 72 and the conical portion 20. A second O-ring 86 is disposed between the cylindrical portion 18 and the base portion 19. Another set of pins, shaft pins 71, connect the cylindrical portion 18 to the base portion 19. The intermediate and shaft pins 70, 71 could comprise any desired number of pins 70, 71, such as three of each set of pins 70, 71. The intermediate and shaft pins 70, 71 are spaced around the circumference of the control section 12. The intermediate pins 70 may be pins or pin-like features that are connected to or unitarily formed with either or both of the conical portion 20 and the shaft bushing 72. Likewise, the shaft pins 71 may be pins or pin-like features that are connected to or unitarily formed with either or both of the cylindrical portion 18 and the base portion 19.

One or more set screws 74 (such as three set screws 74) are spaced around the circumference of the shaft bushing 72. The set screws 74 tighten axially a chassis 76 around the shaft bushing 72, which creates tension between the shaft bushing 72 and the chassis 76. The set screws 74 tighten the chassis 76 around the shaft bushing 72, but they do not fix the chassis 76 to the shaft bushing 72. Instead, the shaft bushing 72 and the conical section 20 rotate over a bearing surface 78 of the chassis 76.

The chassis 76 is connected via a rear housing screw 82 to an extension 84 of the grip portion 16. A shield 85 is connected to the chassis 76 adjacent to the rear housing screw 82. The conical portion 20, the cylindrical portion 18, and the base portion 19 of the control section 12 rotate over the bearing surface 78 of the chassis 76. A third O-ring 88 is disposed between the base portion 19 and the extension 84 of the grip portion 16. A threaded tip 90 is disposed between the insertion tube 14 and the conical portion 20. The threaded tip 90 is screwed onto threads of the shaft bushing 72 and contacts the conical section 20. Accordingly, the shaft bushing 72, the conical section 20, the cylindrical section 18, the base portion 19, and the insertion tube 14 are fixed together, and these components rotate together over the chassis 76.

Accordingly, the control section 12 including the working channel port 22 may be rotated around the chassis 76, which is connected to the grip portion 16, at least 90 degrees in a first direction A and 90 degrees in a second direction B, such that the control section 12 may be rotated a total of at least 180 degrees around the chassis 76.

In some variations, the thin part 60 of the working channel tube 54 has an inner diameter wd of at least 0.4 millimeters The insertion tube 14 second section 28 may have an outer diameter td of 1.8 mm or less, by way of example. In addition, the first section 26 of the insertion tube 14 may have a length in the range of 160-170 mm, by way of example. The passive deflection section 29 of the second section 28 may have a length in the range of 35-40 mm, by way of example. The active deflection section 31 may have a length in the range of about 25-27 mm, by way of example. Thus, the second section 28 has a length of about 60-67 mm in sum, by way of example. Such measurements are preferred when using the endoscope 10 in a sinus cavity.

Referring to FIG. 5, in addition to the control cable 64 and the working channel tube 54, the endoscope 10 may also contain an illumination bundle 92. The illumination bundle 92 may include one or more fiber optic cables, by way of example. The light source post 24 connects a light source (not shown) to the illumination bundle 92. The endoscope may also include an imaging bundle 94 for transmitting an image near the distal end 52 through the imaging bundle 94, which may be viewed by a user through the ocular lens 38, by way of example. The illumination bundle 92 extends through the first and second lumens 48, 50 of the first and section sections 26, 28 of the insertion tube 14 and into the control section 12. The imaging bundle 94 also extends through the first and second lumens 48, 50 of the first and second sections 26, 28 of the insertion tube 14.

## Claims

1. An endoscope (10) for insertion into a small body cavity like a nostril and a sinus, the endoscope (10) comprising:
a control section (12) having portions defining a working channel port (22);
an insertion tube (14) extending from the control section (12), the insertion tube (14) having a first section (26) and a second section (28), the first section (26) defining a longitudinal axis and defining a first lumen (48) extending through the first section (26), the first section (26) having a proximal end (32) connected to the control section (12), the second section (28) defining a second lumen (50) extending through proximal and distal ends (34, 52) of the second section (28), the proximal end (34) of the second section (28) being connected to a distal end (36) of the first section (26), the second section (28) being bendable with respect to the first section (26), the second lumen (50) being in communication with the first lumen (48), wherein the second section (28) includes a passive deflection section (29) and an active deflection section (31), the passive deflection section (29) being connected to and extending from the distal end (36) of the first section (26), the active deflection section (31) extending from the passive deflection section (29) to the distal end (52) of the second section (28);
a working channel tube (54) disposed within the first and second lumens (48, 50), the working channel tube (54) defining a working lumen (56) extending through proximal and a distal ends (62, 63) of the working channel tube (54), the working lumen (56) being in communication with the working channel port (22); and
a grip portion (16) connected to the control section (12), wherein the insertion tube (14) and the control section (12) are rotatable with respect to the grip portion (16),
**characterized in that**
the second section (28) is formed of shape memory alloy;
the endoscope (10) further comprises a wire sheath (68) disposed within the first and second lumens (48, 50), the wire sheath (68) having an interior region and being made of shape memory alloy; and
the endoscope (10) further comprises a single control cable (64) attached to the distal end (52) of the second section (28) of the insertion tube (14), only the single control cable (64) extending through the interior region of the wire sheath (68), the control cable (64) being operable to deflect the active deflection section (31) but not the passive deflection section (29) when the control cable (64) is pulled.

2. The endoscope (10) of claim 1, wherein the insertion tube (14) and the control section (12) are rotatable with respect to the grip portion (16) in a first direction about ninety degrees from a home position and in a second direction about ninety degrees from the home position, the first direction being opposite the second direction.

3. The endoscope (10) of claim 2, wherein the first section (26) of the insertion tube (14) comprises a rigid tube and the second section (28) of the insertion tube (14) comprises a flexible tube.

4. The endoscope (10) of claim 3, wherein the distal end (52) of the second section (28) is configured to be bent from a first position that is located at a first angle from the longitudinal axis to a second position that is located at a second angle from the longitudinal axis, the first and second angles being located on opposite sides of the longitudinal axis, the first angle being at least 30 degrees and the second angle being at least 110 degrees, the second section (28) being bendable along a plane between the first angle and the second angle.

5. The endoscope (10) of claim 4, wherein the second section (28) is preformed into a preformed curved shape, wherein the distal end (52) of the second section (28) extends at an angle of at least 30 degrees from the longitudinal axis in the preformed curved shape.

6. The endoscope (10) of claim 3, further comprising a plurality of pins (70) coupling the control section (12) to the insertion tube (14).

7. The endoscope (10) of claim 6, wherein the working channel tube (54) has an inner diameter of at least 0.4 millimeters.

8. The endoscope (10) of claim 3, further comprising an illumination bundle (92) extending through the first and second lumens (48, 50), the endoscope (10) further comprising an imaging bundle (94) extending through the first and second lumens (48, 50), the endoscope (10) further comprising a lens (38) disposed at a proximal end (40) of the grip portion (16).

9. The endoscope (10) of claim 8, wherein the first section (26) of the insertion tube (14) has a length of about 160-170 millimeters and the second section (28) of the insertion tube (14) has a length of about 60-67 millimeters.

10. An endoscope (10) for insertion into a small body cavity like a nostril and a sinus, the endoscope (10) comprising:
a control section (12) having portions defining a working channel port (22);
an intermediate part (20) connected to the control section (12) by at least one intermediate pin (70);
a shaft bushing (72) connected to the intermediate part (20) by at least one shaft pin (71);
an insertion tube (14) extending from the shaft bushing (72), the insertion tube (14) having a rigid tube section (26) and a flexible tube section (28), the rigid tube section (26) defining a longitudinal axis and defining a first lumen (48) extending through the rigid tube section (26), the rigid tube section (26) having a proximal end (32) connected to the shaft bushing (72), the flexible tube section (28) defining a flexible tube lumen (50) extending through proximal and distal ends (34, 52) of the flexible tube section (28), the proximal end (34) of the flexible tube section (28) being connected to a distal end (26) of the rigid tube section (26), the flexible tube section (28) being bendable with respect to the rigid tube section (26), the flexible tube lumen (50) being in communication with the rigid tube lumen (48), wherein the flexible tube section (28) includes a passive deflection section (29) and an active deflection section (31), the passive deflection section (29) being connected to and extending from the distal end (36) of the rigid tube section (26), the active deflection section (31) extending from the passive deflection section (29) to the distal end (52) of the flexible tube section (28);
a working channel tube (54) disposed within the flexible tube lumen (50) and the rigid tube lumen (48), the working channel tube (54) defining a working tube lumen (56) extending through proximal and distal ends (62, 63) of the working channel tube (54), the proximal end (62) of the working channel tube (54) being connected to the control section (12), the working tube lumen (56) being in communication with the working channel port (22); and
a grip portion (16) connected to the control section (12), wherein the insertion tube (14) and the control section (12) are rotatable with respect to the grip portion (16),
**characterized in that**
the flexible tube section (28) is formed of shape memory alloy;
the endoscope (10) further comprises a wire sheath (68) disposed within the flexible tube lumen (50) and the rigid tube lumen (48), the wire sheath (68) having an interior region and being made of shape memory alloy; and
the endoscope (10) further comprises a single control cable (64) attached to the distal end (52) of the flexible tube section (28) of the insertion tube (14), only the single control cable (64) extending through the interior region of the wire sheath (68), the control cable (64) being operable to deflect the active deflection section (31) but not the passive deflection section (29) when the control cable (64) is pulled.

11. The endoscope (10) of claim 10, wherein the distal end (52) of the flexible tube section (28) is configured to be bent from a first position that is located at a first angle from the longitudinal axis to a second position that is located at a second angle from the longitudinal axis, the first and second angles being located on opposite sides of the longitudinal axis, the first angle being at least 30 degrees and the second angle being at least 110 degrees, the flexible tube section (28) being bendable along a plane between the first angle and the second angle.

12. The endoscope (10) of claim 10, wherein the flexible tube section (28) is preformed into a preformed curved shape, the distal end (52) of the flexible tube section (28) extending at an angle of at least 30 degrees from the longitudinal axis in the preformed curved shape.

13. The endoscope (10) of claim 10, further comprising an illumination bundle (92) extending through the rigid tube lumen (48) and the flexible tube lumen (50), the endoscope (10) further comprising an imaging bundle (94) extending through the rigid tube lumen (48) and the flexible tube lumen (50).

14. The endoscope (10) of claim 12, wherein the insertion tube (14) and the control section (12) are rotatable with respect to the grip portion (16) in a first direction about ninety degrees from a home position and in a second direction about ninety degrees from the home position, the first direction being opposite the second direction.

## Patentansprüche

1. Endoskop (10) zum Einsetzen in einen kleinen Körperhohlraum wie einem Nasenloch und einer Stirnhöhle, wobei das Endoskop (10) umfasst:
einen Steuerabschnitt (12), der Anteile aufweist, die einen Arbeitskanaldurchlass (22) definieren;
ein Einsetzrohr (14), das sich von dem Steuerabschnitt (12) erstreckt, wobei das Einsetzrohr (14) einen ersten Abschnitt (26) und einen zweiten Abschnitt (28) aufweist, der erste Abschnitt (26) eine Längsachse definiert und ein erstes Volumen (48) definiert, das sich durch den ersten Abschnitt (26) erstreckt, der erste Abschnitt (26) ein proximales Ende (32) aufweist, das mit dem Steuerabschnitt (12) verbunden ist, der zweite Abschnitt (28) ein zweites Volumen (50) definiert, das sich durch proximale und distale Enden (34, 52) des zweiten Abschnitts (28) erstreckt, das proximale Ende (34) des zweiten Abschnitts (28) mit einem distalen Ende (36) des ersten Abschnitts (26) verbunden ist, der zweite Abschnitt (28) in Bezug auf den ersten Abschnitt (26) biegbar ist, das zweite Volumen (50) in Kommunikation mit dem ersten Volumen (48) steht, wobei der zweite Abschnitt (28) einen passiven Auslenkabschnitt (29) und einen aktiven Auslenkabschnitt (31) aufweist, wobei der passive Auslenkabschnitt (29) mit dem ersten Abschnitt (26) verbunden ist und sich von dem distalen Ende (36) davon erstreckt, wobei der aktive Auslenkabschnitt (31) sich von dem passiven Auslenkabschnitt (29) zu dem distalen Ende (52) des zweiten Abschnitts (28) erstreckt;
ein Arbeitskanalrohr (54), das in dem ersten und zweiten Volumen (48, 50) angeordnet ist, wobei das Arbeitskanalrohr (54) ein Arbeitsvolumen (56) definiert, das sich durch proximale und distale Enden (62, 63) des Arbeitskanalrohres (54) erstreckt, wobei das Arbeitsvolumen (56) in Kommunikation mit dem Arbeitskanaldurchlass (22) steht; und
einen Greifanteil (16), der mit dem Steuerabschnitt (12) verbunden ist, wobei das Einsetzrohr (14) und der Steuerabschnitt (12) in Bezug auf den Greifanteil (16) drehbar sind,
**dadurch gekennzeichnet, dass**
der zweite Abschnitt (28) aus einer Formgedächtnislegierung geformt ist;
das Endoskop (10) ferner eine Drahtumhüllung (68) umfasst, die in dem ersten und zweiten Volumen (48, 50) angeordnet ist, wobei die Drahtumhüllung (68) einen Innenbereich aufweist und aus einer Formgedächtnislegierung besteht; und
das Endoskop (10) ferner ein einzelnes Steuerseil (64) umfasst, das an dem distalen Ende (52) des zweiten Abschnitts (28) des Einsetzrohres (14) befestigt ist, wobei sich nur das einzelne Steuerseil (64) durch den Innenbereich der Drahtabschirmung (68) erstreckt, wobei das Steuerseil (54) dazu dient, den aktiven Auslenkabschnitt (31) jedoch nicht den passiven Auslenkabschnitt (29) auszulenken, wenn das Steuerseil (64) gezogen ist.

2. Endoskop (10) nach Anspruch 1, wobei das Einsetzrohr (14) und der Steuerabschnitt (12) in Bezug auf den Greifanteil (16) in einer ersten Richtung um etwa neunzig Grad von einer Ausgangsposition und einer zweiten Richtung um etwa neunzig Grad aus der Ausgangsposition drehbar sind, wobei die erste Richtung der zweiten Richtung entgegengesetzt ist.

3. Endoskop (10) nach Anspruch 2, wobei der erste Abschnitt (26) des Einsetzrohres (14) ein starres Rohr umfasst und der zweite Abschnitt (28) des Einsetzrohres (14) ein flexibles Rohr umfasst.

4. Endoskop (10) nach Anspruch 3, wobei das distale Ende (52) des zweiten Abschnitts (28) derart konfiguriert ist, dass es aus einer ersten Position, die unter einem ersten Winkel von der Längsachse angeordnet ist, zu einer zweiten Position gebogen werden kann, die unter einem zweiten Winkel von der Längsachse angeordnet sind, wobei der erste und zweite Winkel auf gegenüberliegenden Seiten der Längsachse angeordnet sind, wobei der erste Winkel zumindest 30 Grad und der zweite Winkel zumindest 110 Grad beträgt, wobei der zweite Abschnitt (28) entlang einer Ebene zwischen dem ersten Winkel und dem zweiten Winkel biegbar ist.

5. Endoskop (10) nach Anspruch 4, wobei der zweite Abschnitt (28) in einer vorgeformten kurvenförmigen Form vorgeformt ist, wobei das distale Ende (52) des zweiten Abschnitts (28) sich unter einem Winkel von zumindest 30 Grad von der Längsachse in der vorgeformten gekrümmten Form erstreckt.

6. Endoskop (10) nach Anspruch 3, ferner mit einer Mehrzahl von Stiften (70), die den Steuerabschnitt (12) mit dem Einsetzrohr (14) koppeln.

7. Endoskop (10) nach Anspruch 6, wobei das Arbeitskanalrohr (54) einen Innendurchmesser von zumindest 0,4 mm aufweist.

8. Endoskop (10) nach Anspruch 3, ferner mit einem Beleuchtungsbündel (92), das sich durch das erste und zweite Volumen (48, 50) erstreckt, wobei das Endoskop (10) ferner ein Bildgebungsbündel (94) umfasst, das sich durch das erste und zweite Volumen (48, 50) erstreckt, wobei das Endoskop (10) ferner eine Linse (38) umfasst, die an einem proximalen Ende (40) des Greifanteiles (16) angeordnet ist.

9. Endoskop (10) nach Anspruch 8, wobei der erste Abschnitt (26) des Einsetzrohres (14) eine Länge von etwa 160 bis 170 mm aufweist und der zweite Abschnitt (28) des Einsetzrohres (14) eine Länge von etwa 60 - 67 Millimeter aufweist.

10. Endoskop (10) zum Einsetzen in einen kleinen Körperhohlraum wie einem Nasenloch oder einer Nebenhöhle, wobei das Endoskop (10) umfasst:
einen Steuerabschnitt (12), der Abschnitte aufweist, die einen Arbeitskanaldurchlass (22) definieren;
ein Zwischenteil (20), das mit dem Steuerabschnitt (12) durch zumindest einen Zwischenstift (70) verbunden ist;
eine Schaftbuchse (72), die mit dem Zwischenteil (20) durch zumindest einen Schaftstift (71) verbunden ist;
ein Einsetzrohr (14), das sich von der Schaftbuchse (72) erstreckt, wobei das Einsetzrohr (14) einen starren Rohrabschnitt (26) und einen flexiblen Rohrabschnitt (28) aufweist, der starre Rohrabschnitt (26) eine Längsachse definiert und ein erstes Volumen (48) definiert, das sich durch den starren Rohrabschnitt (26) erstreckt, der starre Rohrabschnitt (26) ein proximales Ende (32) aufweist, das mit der Schaftbuchse (72) verbunden ist, der flexible Rohrabschnitt (28) ein flexibles Rohrvolumen (50) definiert, das sich durch proximale und distale Enden (34, 52) des flexiblen Rohrabschnitts (28) erstreckt, das proximale Ende (34) des flexiblen Rohrabschnitts (28) mit einem distalen Ende (26) des starren Rohrabschnitts (26) verbunden ist, der flexible Rohrabschnitt (28) in Bezug auf den starren Rohrabschnitt (26) biegbar ist, das flexible Rohrvolumen (50) in Kommunikation mit dem starren Rohrvolumen (48) steht, der flexible Rohrabschnitt (28) einen passiven Auslenkabschnitt (29) und einen aktiven Auslenkabschnitt (31) aufweist, der passive Auslenkabschnitt (29) mit dem starren Rohrabschnitt (26) verbunden ist und sich von dem distalen Ende (36) davon erstreckt, der aktive Auslenkabschnitt (31) sich von dem passiven Auslenkabschnitt (29) zu dem distalen Ende (52) des flexiblen Rohrabschnittes (28) erstreckt;
ein Arbeitskanalrohr (54), das in dem flexiblen Rohrvolumen (50) und dem starren Rohrvolumen (48) angeordnet ist, wobei das Arbeitskanalrohr (54) ein Arbeitsrohrvolumen (56) aufweist, das sich durch proximale und distale Enden (62, 63) des Arbeitskanalrohres (54) erstreckt, das proximale Ende (62) des Arbeitskanalrohres (54) mit dem Steuerabschnitt (12) verbunden ist, das Arbeitsrohrvolumen (56) in Kommunikation mit dem Arbeitskanaldurchlass (22) steht; und
einen Greifanteil (16), der mit dem Steuerabschnitt (12) verbunden ist, wobei das Einsetzrohr (14) und der Steuerabschnitt (12) in Bezug auf den Greifanteil (16) drehbar sind,
**dadurch gekennzeichnet, dass**
der flexible Rohrabschnitt (28) aus Formgedächtnislegierung geformt ist;
das Endoskop (10) ferner eine Drahtumhüllung (68) umfasst, die in dem flexiblen Rohrvolumen (50) und dem starren Rohrvolumen (48) angeordnet ist, wobei die Drahtumhüllung (68) einen Innenbereich aufweist und aus Formgedächtnislegierung besteht; und
das Endoskop (10) ferner ein einzelnes Steuerseil (64) umfasst, das an dem distalen Ende (52) des flexiblen Rohrabschnitts (28) des Einsetzrohres (14) befestigt ist, wobei sich nur das einzelne Steuerseil (64) durch den Innenbereich der Drahtumhüllung (68) erstreckt, wobei das Steuerseil (64) dazu dient, den aktiven Auslenkabschnitt (31), jedoch nicht den passiven Auslenkabschnitt (29) auszulenken, wenn das Steuerseil (64) gezogen wird.

11. Endoskop (10) nach Anspruch 10, wobei das distale Ende (52) des flexiblen Rohrabschnitts (28) derart konfiguriert ist, dass es von einer ersten Position, die unter einem ersten Winkel von der Längsachse angeordnet ist, zu einer zweiten Position biegbar ist, die unter einem zweiten Winkel von der Längsachse angeordnet ist, wobei der erste und zweite Winkel auf entgegengesetzten Seiten der Längsachse angeordnet sind, wobei der erste Winkel zumindest 30 Grad beträgt und der zweite Winkel zumindest 110 Grad beträgt, wobei der flexible Rohrabschnitt (28) entlang einer Ebene zwischen dem ersten Winkel und dem zweiten Winkel biegbar ist.

12. Endoskop (10) nach Anspruch 10, wobei der flexible Rohrabschnitt (28) in eine vorgeformte gekrümmte Form vorgeformt ist, wobei das distale Ende (52) des flexiblen Rohrabschnitts (28) sich unter einem Winkel von zumindest 30 Grad von der Längsachse in der vorgeformten gekrümmten Form erstreckt.

13. Endoskop (10) nach Anspruch 10, ferner mit einem Beleuchtungsbündel (92), das sich durch das starre Rohrvolumen (48) und das flexible Rohrvolumen (50) erstreckt, wobei das Endoskop (10) ferner ein Bildgebungsbündel (94) umfasst, das sich durch das starre Rohrvolumen (48) und das flexible Rohrvolumen (50) erstreckt.

14. Endoskop (10) nach Anspruch 12, wobei das Einsetzrohr (14) und der Steuerabschnitt (12) in Bezug auf den Greifanteil (16) in einer ersten Richtung von etwa neunzig Grad von einer Ausgangsposition und in einer zweiten Richtung von etwa neunzig Grad von der Ausgangsposition drehbar sind, wobei die erste Richtung der zweiten Richtung entgegengesetzt ist.

## Revendications

1. Endoscope (10) destiné à être inséré dans une petite cavité corporelle comme une narine et un sinus, l'endoscope (10) comprenant :
une section de commande (12) ayant des portions définissant un orifice pour canal de travail (22) ;
un tube d'insertion (14) s'étendant depuis la section de commande (12), le tube d'insertion (14) ayant une première section (26) et une seconde section (28), la première section (26) définissant un axe longitudinal et définissant un premier lumen (48) s'étendant à travers la première section (26), la première section (26) ayant une extrémité proximale (32) connectée à la section de commande (12), la seconde section (28) définissant un second lumen (50) s'étendant à travers l'extrémité proximale et l'extrémité distale (34, 52) de la seconde section (28), l'extrémité proximale (34) de la seconde section (28) étant connectée à une extrémité distale (36) de la première section (26), la seconde section (28) étant capable de fléchir par rapport à la première section (26), le second lumen (50) étant en communication avec le premier lumen (48), dans lequel la seconde section (28) inclut une section de déflexion passive (29) et une section de déflexion active (31), la section de déflexion passive (29) étant connectée à et s'étendant depuis l'extrémité distale (36) de la première section (26), la section de déflexion active (31) s'étendant depuis la section de déflexion passive (29) jusqu'à l'extrémité distale (52) de la seconde section (28) ;
un tube de canal de travail (54) disposé dans le premier et dans le second lumen (48, 50), le tube de canal de travail (54) définissant un lumen de travail (56) s'étendant à travers l'extrémité proximale et l'extrémité distale (62, 63) du tube de canal de travail (54), le lumen de travail (56) étant en communication avec l'orifice (22) du canal de travail ; et
une portion formant poignée (16) connectée à la section de commande (12), dans laquelle le tube d'insertion (14) et la section de commande (12) sont capables de rotation par rapport à la portion formant poignée (16),
**caractérisé en ce que**
la seconde section (28) est formée d'un alliage à mémoire de forme ;
l'endoscope (10) comprend en outre une gaine pour câble (68) disposée à l'intérieur du premier et du second lumen (48, 50), la gaine pour câble (68) ayant une région intérieure et étant réalisée en alliage à mémoire de forme ; et
l'endoscope (10) comprend en outre un unique câble de commande (64) attaché à l'extrémité distale (52) de la seconde section (28) du tube d'insertion (14), seul l'unique câble de commande (64) s'étend à travers la région intérieure de la gaine pour câble (68), le câble de commande (64) ayant pour fonction de défléchir la section de déflexion active (31) mais non pas la section de déflexion passive (29) quand le câble de commande (64) est tiré.

2. Endoscope (10) selon la revendication 1, dans lequel le tube d'insertion (14) et la section de commande (12) sont capables de rotation par rapport à la portion formant poignée (16) dans une première direction sur environ 90° depuis une position de repos et dans une seconde direction sur environ 90° depuis la position de repos, la première direction étant opposée à la seconde direction.

3. Endoscope (10) selon la revendication 2, dans lequel la première section (26) du tube d'insertion (14) comprend un tube rigide et la seconde section (28) du tube d'insertion (14) comprend un tube flexible.

4. Endoscope (10) selon la revendication 3, dans lequel l'extrémité distale (52) de la seconde section (28) est configurée pour être fléchie depuis une première position qui est située à un premier angle depuis l'axe longitudinal jusqu'à une seconde position qui est située à un second angle depuis l'axe longitudinal, le premier et le second angle étant situés sur des côtés opposés de l'axe longitudinal, le premier angle étant d'au moins 30° et le second angle étant d'au moins 110°, la seconde section (28) pouvant être fléchie le long d'un plan entre le premier angle et le second angle.

5. Endoscope (10) selon la revendication 4, dans lequel la seconde section (28) est préformée sous une forme incurvée préformée, dans lequel l'extrémité distale (52) de la seconde section (28) s'étend sous un angle d'au moins 30° depuis l'axe longitudinal dans la forme incurvée préformée.

6. Endoscope (10) selon la revendication 3, comprenant en outre une pluralité de broches (70) qui couplent la section de commande (12) au tube d'insertion (14).

7. Endoscope (10) selon la revendication 6, dans lequel le tube de canal de travail (54) a un diamètre intérieur d'au moins 0,4 mm.

8. Endoscope (10) selon la revendication 3, comprenant en outre un faisceau d'éclairage (92) s'étendant à travers le premier et le second lumen (48, 50), l'endoscope (10) comprenant en outre un faisceau d'imagerie (94) s'étendant à travers le premier et le second lumen (48, 50), l'endoscope (10) comprenant en outre une lentille (38) disposée à une extrémité proximale (40) de la portion formant poignée (16).

9. Endoscope (10) selon la revendication 8, dans lequel la première section (26) du tube d'insertion (14) a une longueur d'environ 160 à 170 mm, et la seconde section (28) du tube d'insertion (14) a une longueur d'environ 60 à 67 mm.

10. Endoscope (10) destiné à être inséré dans une petite cavité corporelle comme une narine et un sinus, l'endoscope (10) comprenant :
une section de commande (12) ayant des portions définissant un orifice de canal de travail (22) ;
une partie intermédiaire (20) connectée à la section de commande (12) par au moins une broche intermédiaire (70) ;
une douille à tige (72) connectée à la partie intermédiaire (20) par au moins une tige (71) ;
un tube d'insertion (14) s'étendant depuis la douille à tige (72), le tube d'insertion (14) ayant une section de tube rigide (26) et une section de tube flexible (28), la section de tube rigide (26) définissant un axe longitudinal et définissant un premier lumen (48) s'étendant à travers la section de tube rigide (26), la section de tube rigide (26) ayant une extrémité proximale (32) connectée à la douille à tige (72), la section de tube flexible (28) définissant un lumen de tube flexible (50) s'étendant à travers l'extrémité proximale et l'extrémité distale (34, 52) de la section de tube flexible (28), l'extrémité proximale (34) de la section de tube flexible (28) étant connectée à une extrémité distale (26) de la section de tube rigide (26), la section de tube flexible (28) pouvant être cintrée par rapport à la section de tube rigide (26), le lumen (50) du tube flexible étant en communication avec le lumen du tube rigide (48), dans lequel la section de tube flexible (28) inclut une section de déflexion passive (29) et une section de déflexion active (31), la section de déflexion passive (29) étant connectée à et s'étendant depuis l'extrémité distale (36) de la section de tube rigide (26), la section de déflexion active (31) s'étendant depuis la section de déflexion passive (29) jusqu'à l'extrémité distale (52) de la section de tube flexible (28) ;
un tube de canal de travail (54) disposé à l'intérieur du lumen de tube flexible (50) et du lumen de tube rigide (48), le tube de canal de travail (54) définissant un lumen de tube de travail (56) s'étendant à travers l'extrémité proximale et l'extrémité distale (62, 63) du tube de canal de travail (54), l'extrémité proximale (62) du tube de canal de travail (54) étant connectée à la section de commande (12), le lumen du tube de travail (56) étant en communication avec l'orifice de canal de travail (22) ; et
une portion formant poignée (16) connectée à la section de commande (12), dans lequel le tube d'insertion (14) et la section de commande (12) sont capables de rotation par rapport à la portion formant poignée (16),
**caractérisé en ce que**
la section de tube flexible (28) est formée en alliage à mémoire de forme ;
l'endoscope (10) comprend en outre une gaine pour câble (68) disposée à l'intérieur du lumen (50) du tube flexible et du lumen (48) du tube rigide, la gaine pour câble (68) ayant une région intérieure et étant réalisée en alliage à mémoire de forme ; et
l'endoscope (10) comprend en outre un unique câble de commande (64) attaché à l'extrémité distale (52) de la section de tube flexible (28) du tube d'insertion (14), seul l'unique câble de commande (64) s'étendant à travers la région intérieure de la gaine pour câble (68), le câble de commande (64) ayant pour fonction de défléchir la section de déflexion active (31) mais non pas la section de déflexion passive (29) quand le câble de commande (64) est tiré.

11. Endoscope (10) selon la revendication 10, dans lequel l'extrémité distale (52) de la section de tube flexible (28) est configurée pour être cintrée depuis une première position qui est située à un premier angle depuis l'axe longitudinal jusqu'à une seconde position qui est située à un second angle depuis l'axe longitudinal, le premier et le second angle étant situés sur des côtés opposés de l'axe longitudinal, le premier angle étant d'au moins 30° et le second angle étant d'au moins 110°, la section de tube flexible (28) pouvant être cintrée le long d'un plan entre le premier angle et le second angle.

12. Endoscope (10) selon la revendication 10, dans lequel la section de tube flexible (28) est préformée sous une forme incurvée préformée, l'extrémité distale (52) de la section de tube flexible (28) s'étendant sous un angle d'au moins 30° depuis l'axe longitudinal dans la forme incurvée préformée.

13. Endoscope (10) selon la revendication 10, comprenant en outre un faisceau d'éclairage (92) s'étendant à travers le lumen de tube rigide (48) et le lumen de tube flexible (50), l'endoscope (10) comprenant en outre un faisceau d'imagerie (94) qui s'étend à travers le lumen de tube rigide (48) et le lumen de tube flexible (50).

14. Endoscope (10) selon la revendication 12, dans lequel le tube d'insertion (14) et la section de commande (12) sont capables de rotation par rapport à la portion formant poignée (16) dans une première direction sur environ 90° depuis une position de repos, et dans une seconde direction sur environ 90° depuis la position de repos, la première direction étant opposée à la seconde direction.
